# EUROPÄISCHE PATENTSCHRIFT

(11) **EP 0 976 716 B1**
(45) Veröffentlichungstag und Bekanntmachung des Hinweises auf die Patenterteilung: **19.02.2003**
(21) Anmeldenummer: 99114131.8
(22) Anmeldetag: 19.07.1999
(51) Int. Cl.: C07C 67/60, C07C 69/54

(54) **Verfahren zur Herstellung von Estern der (Meth)acrylsäure**
Process for the preparation of esters of (meth)acrylic acid
Procédé de préparation d'esters d'acide (méth)acrylique

(30) Priorität: 30.07.1998 DE 19834360
(43) Veröffentlichungstag der Anmeldung: 02.02.2000
(73) Patentinhaber: BAYER AG, 51368 Leverkusen (DE)
(72) Erfinder: Weikard, Jan, Dr., 50670 Köln (DE); Fischer, Wolfgang, Dr., 40668 Meerbusch (DE)

(56) Entgegenhaltungen:
- EP-A- 0 279 303
- EP-A- 0 686 621

## Beschreibung

Die Erfindung betrifft ein Verfahren zur Herstellung von Estern der Propensäure und der 2-Methylpropensäure ((Meth)acrylsäureester).

Ester der (Meth)acrylsäure, z.B. der (2-Methyl-)Propensäure, finden in der Beschichtungstechnologie Anwendung zur Herstellung von durch energiereiche Strahlung härtbaren Bindemitteln. Diese sollen einen möglichst geringen Gehalt an freier (2-Methyl-)Propensäure aufweisen.

Es ist bekannt, daß sich Hydroxylgruppen aufweisende Polyester mit (2-Methyl-)-Propensäure (Methacrylsäure) sauer katalysiert azeotrop verestern lassen. Dabei wird nach Entfernung des Lösungsmittels nicht umgesetzte (2-Methyl-)Propensäure mit Epoxiden (Oxiranverbindungen) chemisch gebunden (z.B. EP-A 54 105, 126 341). Diese Verfahren besitzen folgende Nachteile. In dem in der EP-A 54 105 beschriebenen Verfahren liegt die eingesetzte (Meth)acrylsäure nur im Unterschuß vor (max. 90 Mol-%, bezogen auf die OH-Gruppen des Polyesters), so daß die entstehenden Produkte aufgrund des Restgehalts an Hydroxylgruppen unerwünscht erhöhte Viskositäten aufweisen. Das in der EP-A 126 341 beschriebene Verfahren erfordert zusätzlich vor Abtrennung nicht veresterter (Meth)acrylsäure mit Epoxiden die Neutralisation des Veresterungskatalysators mit Alkalihydroxiden oder tert. Aminen.

Weiterhin ist aus der EP-A 686621 ein Verfahren bekannt, bei dem in einer zweiten Stufe mit einer Epoxidverbindung in Gegemwart von quarternären Ammonium- oder Phosphoniumverbindungen umgesetzt wird.

Der Verbleib von niedermolekularen, nicht einpolymerisierenden Verbindungen aus Veresterungskatalysator und Neutralisationsmittel im (Meth)acrylsäureester ist nachteilig für Härte und Beständigkeit der damit herzustellenden Beschichtungen. Weiterhin greifen tert. Amine in die UV-Härtung entsprechender Bindemittelkombinationen ein (P.K.T. Oldring, Chemistry & Technology of UV & EB Formulations for Coatings, Ink & Paints, 1991, S. 192-196), so daß ihre Anwesenheit in den Rohstoffen für Beschichtungen unerwünscht ist.

Bei den obengenannten Verfahren ist von Nachteil, daß zur Umsetzung der nicht veresterten (Meth)acrylsäure mit Epoxid-haltigen Verbindungen das Lösungsmittel zunächst destillativ entfernt werden muß und dann erst die Umsetzung mit Epoxiden bei erhöhten Temperaturen bis 130°C durchgeführt wird. Diese hohen Temperaturen sind unerwünscht, da z.B. eine spontane ungewollte Polymerisation der (Meth)acrylsäure eintreten kann, deren technische Beherrschung aufgrund der fehlenden Siedekühlung durch das Lösungsmittel erschwert wird.

Es war daher wünschenswert, ein Verfahren zur Herstellung von (Meth)acrylsäureestern (Estern der (2-Methyl-)Propensäure) zu finden, das die aufgeführten Nachteile des Standes der Technik nicht aufweist.

Es wurde nun gefunden, daß sich bei der Veresterung Hydroxylgruppen-haltiger Polyester oder Polyether mit (Meth)acrylsäure im Anschluß an die Veresterung sowohl die Katalysatorsäure als auch nicht umgesetzte (Meth)acrylsäure im Lösungsmittel der Veresterung mit zwei- oder mehrwertigen Oxiranverbindungen (Epoxiden) bis zu einer Säurezahl von kleiner oder gleich 5 (mg KOH/g Substanz) umsetzen lassen, wobei im Anschluß an diese Umsetzung das Lösungsmittel unter vermindertem Druck (bis 10⁻⁵ bar) destillativ aus dem Reaktionsgemisch entfernt wird. Eine thermische Belastung der konzentrierten (Meth)acrylsäureester bleibt gering, so daß eine spontane, unerwünschte Polymerisation der hergestellten (Meth)acrylsäureester weitestgehend vermieden wird.

Gegenstand der Erfindung ist demnach ein Verfahren zur Herstellung von (Meth)acrylsäureestern aus Hydroxylgruppen aufweisenden Polyethern und/oder aus Hydroxylgruppen aufweisenden, gegebenenfalls Ethergruppen enthaltenden Polyestern durch säurekatalysierte Veresterung in einem Lösungsmittel, das mit Wasser nicht mischbar ist und mit Wasser im Sinne einer Wasserdampf-Destillation destillierbar ist, dadurch gekennzeichnet, dass die Katalysatorsäure der Veresterungsreaktion sowie nicht umgesetzte (Meth)acrylsäure in der Reaktionslösung mit (Oligo)Epoxiden ohne Zusatz von weiteren Katalysatoren umgesetzt werden, und das Lösungsmittel anschließend unter vermindertem Druck destillativ aus dem Reaktionsgemisch entfernt wird.

Für das erfindungsgemäße Verfahren kann Acrylsäure (Propensäure) oder Methacrylsäure (2-Methylpropensäure) oder ein beliebiges Gemisch aus beiden eingesetzt werden.

Für das erfindungsgemäße Verfahren werden Polyester eingesetzt, deren Alkoholkomponente von ein- oder mehrwertigen, gesättigten, aliphatischen oder cycloaliphatischen C₁-C₂₀-Alkoholen stammt, die gegebenenfalls Ethergruppen enthalten. Solche Alkohole haben Molekulargewichte von 32 bis etwa 800. Beispielhaft seien folgende Alkohole genannt: Methanol, Ethanol, Propanole, Butanole, Hexanole, Cetylalkohol, Stearylalkohol, Ethylenglykol, Propylenglykol, Butandiol-1,4, Pentandiol-1,5, Neopentylglykol, Hexandiol-1,6, Glycerin, Trimethylolethan, Trimethylolpropan, Pentaerythrit, 2-Ethylhexanol, Cyclohexanol, Dimethylolcyclohexan sowie Oxalkylierungsprodukte der genannten Alkohole mit beispielsweise 1 bis 5 Mol Ethylenoxid oder Propylenoxid pro Hydroxyl-Äquivalent. Die Säurekomponente dieser Polyester stammt von gesättigten C₁-C₂₀-aliphatischen oder C₆-C₂₄-aromatischen Carbonsäuren ab wie Monocarbonsäuren, z.B. Benzoesäure, Dicarbonsäuren wie z.B. Bernsteinsäure, Glutarsäure, Adipinsäure, Phthal-, Isophthal- und Terephthalsäure, substituierte Phthalsäuren. Die entsprechenden Säureanhydride sind ebenfalls einsetzbar.

Als saure Veresterungskatalysatoren werden im erfindungsgemäßen Verfahren anorganische oder organische Säuren in einer Menge von 0,1 bis 3 Gew.-%, bezogen auf das Gewicht der zu veresternden (Meth)acrylsäure, eingesetzt. Beispiele für solche Veresterungskatalysatoren sind Schwefelsäure, Phosphorsäure, Pyrophosphorsäure, p-Toluolsulfonsäure, Styroldivinylbenzolsulfonsäure, Chlorsulfonsäure, Chlorameisensäure, bevorzugt Schwefelsäure und p-Toluolsulfonsäure.

Das erfmdungsgemäße Verfahren wird in einem Lösungsmittel durchgeführt, das mit Wasser nicht mischbar ist und mit Wasser im Sinne einer Wasserdampf-Destillation destillierbar ist. Hierfür kommen Kohlenwasserstoffe sowie deren Halogen- oder Nitro-Substitutionsprodukte in Betracht sowie weitere Lösungsmittel, die weder mit den Reaktionspartnern reagieren noch sich unter dem Einfluß der sauren Katalysatoren verändern. In bevorzugter Weise werden unsubstituierte Kohlenwasserstoffe eingesetzt. Beispielhaft seien genannt: aliphatische Kohlenwasserstoffe, wie Hexan, Heptan, Octan, Benzinfraktionen verschiedener Siedebereiche, cycloaliphatischer Kohlenwasserstoff, wie Cyclopentan, Cyclohexan, Methylcyclohexan, oder aromatische Kohlenwasserstoffe wie Benzol, Toluol oder die isomeren Xylole. In bevorzugter Weise werden solche Lösungsmittel eingesetzt, die im Bereich von 70 bis 120°C sieden. Insbesondere seien hier Cyclohexan, Toluol oder Benzinfraktionen im Siedebereich von 70 bis 120°C genannt. Das mit Wasser nicht mischbare Lösungsmittel kann auch ein Gemisch der obengenannten Stoffe sein. Es wird in einer Menge von 10 bis 100 Gew.-%, bevorzugt 15 bis 50 Gew.-%, besonders bevorzugt 20 bis 40 Gew.-%, bezogen auf das Gewicht der zu veresternden Reaktionskomponenten, eingesetzt.

Zur erfindungsgemäßen Herstellung der (Meth)acrylsäureester aus Hydroxylgruppen haltigen Polyestern wird dem Reaktionsschritt in Lösungsmittel eine lösungsmittelfreie Schmelzkondensation der beschriebenen Alkoholkomponenten mit den beschriebenen gesättigten bzw. aromatisch ungesättigten Carbonsäuren vorangestellt und dieses so erhaltene Prekondensat mit (Meth)acrylsäure weiter in einem der beschriebenen Lösungsmittel verestert. Es ist aber auch möglich die Reaktion der beschriebenen Alkoholkomponenten mit den beschriebenen gesättigten bzw. aromatisch ungesättigten Carbonsäuren im gleichen Lösungsmittel durchzuführen. Das Reaktionswasser wird dann vom siedenden Lösungsmittel aus dem Reaktionsgemisch geschleppt.

Das erfindungsgemäße Verfahren kann in Gegenwart eines oder mehrerer Polymerisationsinhibitoren in einer Menge von 0,01 bis 1 Gew.-%, bevorzugt 0,1 bis 0,5 Gew.-%, bezogen auf das zu veresternde Gemisch aus (2-Methyl-)Propensäure und Hydroxylgruppen-haltige Verbindung, durchgeführt werden. Solche Inhibitoren sind beispielsweise in Houben-Weyl, Methoden der organischen Chemie, 4. Auflage, Band XIV/1, Georg Thieme Verlag, Stuttgart 1961, Seite 433 ff. beschrieben. Als Beispiele seien genannt: Natriumdithionit, Natriumhydrogensulfit, Schwefel, Hydrazin, Phenylhydrazin, Hydrazobenzol, N-Phenyl-β-naphthylamin, N-Phenyl-ethanolamin, Dinitrobenzol, Picrinsäure, p-Nitroso-dimethylanilin, Diphenylnitrosamin, Phenole, wie p-tert.-Butyl-brenzcatechin, 2,5-Di-tert.-amylhydrochinon, p-Alkoxyphenole, Di-tert.-butylhydrochinon, Tetramethyl-thiuramdisulfid, 2-Mercaptobenzthiazol und Dimethyl-dithiocarbaminsäure-Natriumsalz.

Weiterhin wird in einer bevorzugten Variante des erfindungsgemäßen Verfahrens ein sauerstoffhaltiges Gas, vorzugsweise Luft, in das lösungsmittelhaltige Reaktionsgemisch eingeleitet.

Entsprechend dem erfindungsgemäßen Verfahren wird zunächst die Veresterung der (2-Methyl-)Propensäure in einem Temperaturbereich von 60 bis 140°C, bevorzugt 70 bis 120°C, besonders bevorzugt beim Siedepunkt des eingesetzten Lösungsmittels, durchgeführt. Hierbei wird ständig Lösungsmittel aus dem Reaktionsgemisch destillativ abgezogen, außerhalb des Reaktionsgefäßes in einem Wasserabscheider von herausgeschlepptem Wasser abgetrennt und danach wieder in das Reaktionsgemisch zurückgeführt. Das Ende der Reaktion ist erreicht, wenn eine dem gewünschten Umsetzungsgrad der Reaktion entsprechende Menge Wasser abgetrennt wurde oder die Säurezahl des Reaktionsgemischs auf einen, dem gewünschten Umsetzungsgrad entsprechenden Wert gefallen ist. Bezogen auf die OH-Gruppen der Hydroxylgruppen-haltigen Verbindung entspricht dies einem Umsetzungsgrad von 60 bis 100 %, bevorzugt 70 bis 89 %, besonders bevorzugt von 75 bis 84 % im erfindungsgemäßen Verfahren.

Nach beendeter Veresterung erfolgt ohne weitere Zwischenschritte, wie beispielsweise Neutralisation des Katalysators oder Abdestillieren des Lösungsmittels, die Umsetzung mit (Oligo)epoxiden, z.B. mit zwei- oder mehrwertigen Epoxid(Oxiran)-verbindungen. Diese wird bei Temperaturen von 50 bis 129°C, bevorzugt von 70 bis 99°C, besonders bevorzugt bei 80 bis 89°C, durchgeführt.

Beispiele für erfindungsgemäß einsetzbare (Oligo)epoxide sind zwei- oder mehrwertige Epoxide (Oxiranverbindungen) wie die Glycidylether bzw. -ester aliphatischer oder aromatischer Polyole bzw. Polysäuren wie z.B. Hexandiolbisglycidylether, Bisphenol-A-bisglycidylether, Hexahydrophthalsäurebisglycidylester, Glycerintriglycidylether, Pentaerythrittriglycidylether.

Die erfindungsgemäß einzusetzenden Epoxide werden im Verhältnis Oxirangruppen zu Säuregruppen (aus Katalysatorsäure und nicht veresterter (2-Methyl-)Propensäure, durch Ermittlung der Säurezahl des Reaktionsgemischs bestimmbar) von 2:1 bis 1:1 mol, bevorzugt jedoch im Verhältnis 1,4:1 bis 1,1:1 mol, besonders bevorzugt im Verhältnis 1,2:1 mol, zugegeben.

Die Umsetzung der Epoxide mit der Katalysatorsäure und nicht veresterter (Meth) acrylsäure erfolgt nicht in Gegenwart von weiteren Katalysatoren also ohne zusatz von weiteren katalysatoren.

Die Umsetzung erfolgt so lange, bis die Säurezahl auf den Wert von kleiner 5 (mg KOH/g Substanz) gefallen ist.

Die erfindungsgemäß hergestellten (Meth)acrylsäureester finden Verwendung als Bindemittel in strahlenhärtenden oder konventionell härtenden Spachtel- und Überzugsmassen.

### Beispiele

### Beispiel 1

### Ethergruppen-haltiger Acrylsäureester (Propensäureester)

775 g eines Triols, hergestellt aus Trimethylolpropan und 4 mol Ethylenoxid, und 468 g Propensäure werden zusammen mit 1,5 Gew.-% (bezogen auf die Summe aus Polyol und Propensäure), p-Toluolsulfonsäure, sowie mit 3 000 ppm p-Methoxyphenol und 200 ppm 2,5-Di-tert.-Butyl-hydrochinon eingewogen und unter Rühren mit 400 g Isooctan gemischt. Unter Durchleitung von Luft (einfaches Kesselvolumen pro Stunde) und Überleitung von Stickstoff (doppeltes Kesselvolumen pro Stunde) wird unter andauerndem Rühren auf Rückflußtemperatur aufgeheizt (ca. 100°C). Das entstehende Reaktionswasser wird abgeschieden und der Rückfluß so lange aufrecht erhalten, bis eine Säurezahl von ca. 5 (mg KOH/g Substanz) erreicht ist. Die Apparatur wird auf 85°C gekühlt, bei 85°C werden 15 Bisphenol-A-diglycidylether zugegeben, unter Durchleiten von Luft und Überleiten von Stickstoff (Bedingungen siehe oben) 2 Stunden bei 85°C gehalten. Danach wird das Lösungsmittel unter vermindertem Druck abdestilliert. Der entstandene Propensäureester ist klar, hat eine Säurezahl von <1 (mg KOH/g Substanz) und eine Viskosität von 164 mPa.s bei 23°C.

### Beispiel 2 (außerhalb der Erfindung)

### Estergruppen-haltiger Acrylsäureester (Propensäureester)

Es werden zur Herstellung eines Polyesters durch Schmelzkondensation 321 g Adipinsäure und 1616 g eines Triols, hergestellt aus Trimethylolpropan und 4 mol Ethylenoxid, unter Überleiten von Stickstoff (einfaches Kesselvolumen pro Stunde) bei 145°C aufgeschmolzen und 4 Stunden bei dieser Temperatur gerührt, wobei entstehendes Reaktionswasser abdestilliert. Anschließend wird die Temperatur auf 190°C gesteigert und gehalten, bis eine Säurezahl von kleiner 5 (mg KOH/g Substanz) erreicht ist.

Vom so hergestellten Polyester werden 760 g zusammen mit 363 g Acrylsäure (Propensäure) und 1,0 Gew.-% (bezogen auf die Summe aus Polyester und Propensäure) p-Toluolsulfonsäure, sowie mit 3 000 ppm p-Methoxyphenol und 200 ppm 2,5-Di-tert.-butyl-hydrochinon eingewoben und unter Rühren mit 450 g Isooctan gemischt. Unter Durchleitung von Luft (einfaches Kesselvolumen pro Stunde) und unter gleichzeitiger Überleitung von Stickstoff (doppeltes Kesselvolumen pro Stunde) wird unter andauerndem Rühren auf Rückflußtemperatur aufgeheizt (ca. 100°C). Das entstehende Reaktionswasser wird abgeschieden und der Rückstand so lange bei dieser Temperatur gehalten, bis eine Säurezahl von ca. 40 (mg KOH/g Substanz) erreicht ist. Bei 90°C werden 193 g Bisphenol-A-diglycidylether und als Katalysator 0,5 Gew.-% (bezogen auf Festgehalt) Triethylbenzylammoniumchlorid zugegeben und unter Durchleiten von Luft und Überleiten von Stickstoff (Bedingungen siehe oben) bei 90°C gehalten, bis eine Säurezahl von kleiner 5 (mg KOH/g Substanz) erreicht ist. Danach wird das Lösungsmittel unter vermindertem Druck abdestilliert. Der entstandene Acryl(Propen)säureester ist klar und hat eine Viskosität von 1 700 mPa.s bei 23°C.

### Vergleichsbeispiel

Vom im Beispiel 2 hergestellten Polyester werden 760 g zusammen mit 363 g Acrylsäure (Propensäure) und 1,0 Gew.-% (bezogen auf die Summe aus Polyester und Propensäure) p-Toluolsulfonsäure, sowie mit 3 000 ppm p-Methoxyphenol und 200 ppm 2,5-Di-tert.-butyl-hydrochinon eingewogen und unter Rühren mit 450 g Cyclohexan gemischt. Unter Durchleitung von Luft (einfaches Kesselvolumen pro Stunde) und unter gleichzeitiger Überleitung von Stickstoff (doppeltes Kesselvolumen pro Stunde) wird unter andauerndem Rühren auf Rückflußtemperatur aufgeheizt (ca. 85°C). Das entstehende Reaktionswasser wird abgeschieden und der Rückfluß so lange aufrecht erhalten, bis eine Säurezahl von ca. 40 (mg KOH/g Substanz) erreicht ist. Der Katalysator wird durch Zugabe einer äquivalenten Menge N,N-Dimethyl-ethanolamin neutralisiert. Danach wird das Lösungsmittel unter vermindertem Druck abdestilliert. Bei 90°C werden 193 g Bisphenol-A-diglycidylether und als Katalysator 0,5 Gew.-% (bezogen auf Festgehalt) Triethylbenzylammoniumchlorid zugegeben und unter Durchleiten von Luft und unter gleichzeitigem Überleiten von Stickstoff (Bedingungen siehe oben) bei 90°C gehalten, bis eine Säurezahl von kleiner 5 (mg KOH/g Substanz) erreicht ist. Der entstandene Acryl-(Propen)säureester ist klar und hat eine Viskosität von 1 400 mPa.s bei 23°C.

## Patentansprüche

1. Verfahren zur Herstellung von (Meth)acrylsäureestem aus Hydroxylgruppen aufweisenden Polyethern und/oder aus Hydroxylgruppen aufweisenden, gegebenenfalls Ethergruppen enthaltenden Polyestern durch säurekatalysierte Veresterung in einem Lösungsmittel, das mit Wasser nicht mischbar ist und mit Wasser im Sinne einer Wasserdampf-Destillation destillierbar ist, **dadurch gekennzeichnet, dass** die Katalysatorsäure der Veresterungsreaktion sowie nicht umgesetzte (Meth)acrylsäure in der Reaktionslösung mit (Oligo)Epoxiden ohne Zusatz von weiteren Katalysatoren umgesetzt werden, und das Lösungsmittel anschließend unter vermindertem Druck destillativ aus dem Reaktionsgemisch entfernt wird.

2. Verfahren nach Anspruch 1, **dadurch gekennzeichnet, daß** die Umsetzung des sauren Veresterungskatalysators und nicht veresterter (Meth)acrylsäure im Lösungsmittel der Veresterung durch Reaktion mit (Oligo)Epoxiden bei Temperaturen von 70 bis 99°C durchgeführt wird.

3. Verfahren nach Anspruch 1, **dadurch gekennzeichnet, daß** die Veresterung der Hydroxylgruppen aufweisenden, gegebenenfalls Ethergruppen enthaltenden Polyester mit (Meth)acrylsäure bis zu einem auf OH-Gruppen bezogenen Umsatz von 70 bis 89 % durchgeführt wird.

4. Verfahren nach Anspruch 1, **dadurch gekennzeichnet, daß** als (Oligo)Epoxid Bisphenol-A-diglycidylether eingesetzt wird.

## Claims

1. A process for preparing (meth)acrylates from hydroxyl group-containing polyethers and/or from hydroxyl group-containing polyesters which optionally also contain ether groups by acid-catalysed esterification in a solvent which is not miscible with water and can be distilled with water in the context of a steam distillation, **characterised in that** the catalyst acid from the esterification reaction and unreacted (meth)acrylic acid are reacted with (oligo)epoxides, in the reaction solution, without adding further catalysts, and the solvent is then removed from the reaction mixture by distillation under reduced pressure.

2. A process according to Claim 1, **characterised in that** reaction of the acid esterification catalyst and non-esterified (meth)acrylic acid is performed by reacting with (oligo)epoxides at temperatures of 70 to 99°C in the solvent for esterification.

3. A process according to Claim 1, **characterised in that** esterification of the hydroxyl group-containing polyesters which optionally also contain ether groups with (meth)acrylic acid is performed up to a conversion of 70 to 89 %, with respect to the OH groups.

4. A process according to Claim 1, **characterised in that** bisphenol-A diglycidyl ether is used as an (oligo)epoxide.

## Revendications

1. Procédé de préparation d'esters d'acide (méth)acrylique à partir de polyéthers présentant des radicaux hydroxyle et/ou de polyesters, contenant le cas échéant des radicaux éther, présentant des radicaux hydroxyle, par estérification en catalyse acide dans un solvant qui n'est pas miscible à l'eau et qui peut être distillé avec de l'eau dans le sens d'un entraînement à la vapeur, **caractérisé en ce que** le catalyseur acide de la réaction d'estérification, ainsi que l'acide (méth)acrylique n'ayant pas réagi sont transformés dans la solution de réaction avec des (oligo)époxydes, sans addition d'autre catalyseur, et le solvant est ensuite éliminé du mélange réactionnel par distillation sous pression réduite.

2. Procédé suivant la revendication 1, **caractérisé en ce que** la réaction du catalyseur acide d'estérification et de l'acide (méth)acrylique n'ayant pas réagi est réalisée dans le solvant de l'estérification, par réaction avec des (oligo)époxydes à des températures allant de 70 à 99°C.

3. Procédé suivant la revendication 1, **caractérisé en ce que** l'estérification du polyester, contenant le cas échéant des radicaux éther, présentant des radicaux hydroxyle, avec l'acide (méth)acrylique est réalisée jusqu'à une transformation sur base des radicaux OH, allant de 70 à 89%.

4. Procédé suivant la revendication 1, **caractérisé en ce que** l'on met en oeuvre comme (oligo)époxyde, le bisphénol-A-diglycidyléther.
